(19) Europäisches Patentamt
European Patent Office
Office européen des brevets

(11) EP 0 652 210 B1

(12) FASCICULE DE BREVET EUROPEEN

(45) Date de publication et mention de la délivrance du brevet:
**13.08.1997 Bulletin 1997/33**

(51) Int Cl.6: **C07C 323/60**, C07D 249/20, A61K 7/42

(21) Numéro de dépôt: **94402539.4**

(22) Date de dépôt: **09.11.1994**

# (54) Nouveaux filtres solaires fluorés hydrocarbonés, leur procédé de préparation et leur utilisation dans des compositions cosmétiques

Fluorwasserstoffgruppe enthaltende Sonnenfilter, Verfahren zu ihrer Herstellung, und ihre Verwendung in kosmetischen Zusammensetzungen

Solar filters containing a fluorohydrocarbon group, process for their preparation, and their use in cosmetic compositions

(84) Etats contractants désignés:
**AT BE CH DE ES FR GB IT LI NL SE**

(30) Priorité: **10.11.1993 FR 9313462**

(43) Date de publication de la demande:
**10.05.1995 Bulletin 1995/19**

(73) Titulaire: **L'OREAL**
**75008 Paris (FR)**

(72) Inventeurs:
- **Bollens, Eric**
**F-94410 Saint-Maurice (FR)**
- **Mahieu, Claude**
**F-75017 Paris (FR)**

(74) Mandataire: **Casalonga, Axel et al**
**BUREAU D.A. CASALONGA - JOSSE**
**Morassistrasse 8**
**80469 München (DE)**

(56) Documents cités:
**FR-A- 2 237 912**
**FR-A- 2 601 365**
**FR-A- 2 619 811**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 652 210 B1

## Description

La présente invention est relative à de nouveaux composés fluorés hydrocarbonés utilisés à titre de filtres solaires dans des compositions cosmétiques pour la protection de la peau et des cheveux contre le rayonnement ultraviolet, et à leur procédé de préparation.

Les radiations lumineuses de longueurs d'onde comprises entre 280 nm et 400 nm permettent le brunissement de l'épiderme humain.

Or, il est bien connu que les rayons de longueurs d'onde comprises entre 280 et 320 nm, appelés UV-B, provoquent des érythèmes et des brûlures cutanées qui peuvent nuire au développement du bronzage.

Toutefois, si les rayons UV-B de longueurs d'onde comprises entre 280 et 320 nm jouent un rôle prépondérant dans la production d'érythèmes solaires et doivent être filtrés, il n'en est pas moins vrai que les rayonnements UV-A de longueurs d'onde comprises entre 320 et 400 nm provoquant le brunissement de la peau, provoquent également une altération de celle-ci, notamment dans le cas d'une peau sensible ou d'une peau continuellement exposée aux rayonnements solaires. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques.

Par conséquent, on a recherché des composés susceptibles d'absorber aussi bien les rayons UV-A que les rayons UV-B nocifs pour la peau.

Dans ce but, il a été proposé de nombreuses substances qui, pour la plupart, sont des composés aromatiques présentant une absorption des rayons UV dans la zone comprise entre 280 et 315 nm, dans la zone comprise entre 315 et 400 nm ou dans l'ensemble de ces deux zones.

Outre leur pouvoir filtrant, ces composés antisolaires doivent également avoir de bonnes propriétés cosmétiques dans les formulations, une bonne solubilité dans les solvants usuels, ainsi qu'une bonne résistance à l'eau et à la transpiration.

Parmi les composés aromatiques qui ont été préconisés à cet effet, on peut citer les dérivés de l'acide p-aminobenzoïque, les dérivés du benzylidènecamphre, les dérivés de l'acide cinnamique, etc... Cependant, certaines de ces substances ne présentent pas toutes les propriétés requises pour leur utilisation comme filtres UV dans les compositions filtrantes. Leur pouvoir filtrant peut être insuffisant, leur solubilité dans les différents types de formulations utilisés en matière de protection solaire n'est pas toujours suffisamment bonne et elles présentent également une mauvaise résistance à l'eau et à la sueur. Il est également souhaitable que les composés filtrants ne pénètrent pas dans la peau.

Afin de remédier à ces inconvénients, il a été proposé, notamment dans les demandes françaises 2.237.912 et 2.601.365 de la demanderesse, de fixer les composés absorbant les rayons ultraviolets sur les chaînes macromoléculaires de certains polymères. Toutefois, afin d'obtenir des propriétés filtrantes satisfaisantes, il est parfois nécessaire d'utiliser une grande quantité de ces polymères, ce qui se traduit par de mauvaises propriétés cosmétiques des formulations.

La demanderesse a découvert, de façon surprenante, ce qui fait l'objet de la présente invention, de nouveaux composés résultant de l'association d'une ou de plusieurs molécules absorbant les rayons UV, entre 280 et 400 nm, et d'une chaîne perfluorocarbonée, qui présentent de bonnes propriétés filtrantes, une bonne résistance à l'eau et à la sueur, une bonne solubilité dans les solvants usuels et des propriétés de non-pénétration améliorées, les rendant particulièrement appropriés à une utilisation comme filtres solaires dans des compositions cosmétiques. En outre, les compositions cosmétiques contenant ces composés confèrent à la peau ou aux cheveux une agréable douceur.

La présente invention a donc pour objet de nouveaux composés associant une chaîne perfluorocarbonée à une ou plusieurs molécules absorbant dans le domaine des rayons UV-A et UV-B, ainsi que leur procédé de préparation.

Un autre objet de l'invention est constitué par l'utilisation de ces composés à titre de filtres solaires absorbant les rayons UV-A et UV-B, ainsi que par des compositions cosmétiques contenant ces composés à titre de filtres solaires.

L'invention a également pour objet un procédé de protection de l'épiderme humain et des cheveux contre le rayonnement ultraviolet.

D'autres objets apparaîtront à la lecture de la description et des exemples qui suivent.

Les composés nouveaux, conformes à l'invention, répondent à la formule (I) suivante :

$$R_F\text{-}(CH_2)_n\text{-}S\text{-}(CH_2\text{-}\underset{\displaystyle X}{\underset{|}{C}}H\text{-})_p\text{ - }H \qquad (I)$$

dans laquelle :

$R_F$ est un radical perfluoroalkyle, linéaire ou ramifié, en $C_4$-$C_{10}$, de préférence en $C_6$-$C_8$, ou un mélange de radicaux perfluoroalkyle, linéaires ou ramifiés, en $C_4$-$C_{10}$, de préférence en $C_6$-$C_8$;
n est un nombre entier compris entre 0 et 4, de préférence égal à 2;
p est un nombre entier ou statistique, compris entre 1 et 10, de préférence compris entre 1 et 5;
X est un groupement aromatique conférant au composé de formule (I) une absorption de l'ultraviolet dans la zone de longueurs d'onde pouvant aller de 280 à 400 nm.

De préférence, X est le groupement

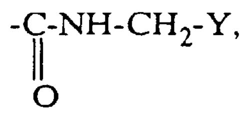

dans lequel Y est un radical aromatique absorbant le rayonnement UV dans le domaine de longueurs d'onde pouvant aller de 280 à 400 nm.

De façon plus particulièrement préférée, Y est choisi parmi les groupements suivants :

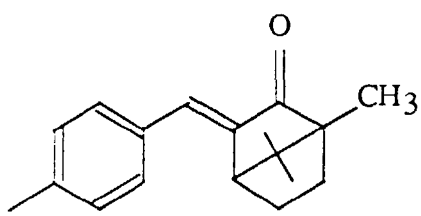

(II)

et

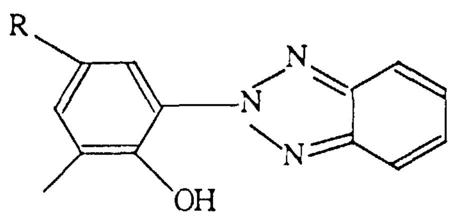

(III)

R désignant le radical méthyle ou tert.-octyle.

Les composés selon l'invention peuvent être préparés par addition radicalaire d'un mercaptan comportant une chaîne perfluoroalkyle, sur une ou plusieurs molécules absorbant le rayonnement UV dans le domaine de longueurs d'onde choisi et comportant un groupe contenant une double liaison réactive ne participant pas à l'absorption UV, selon le schéma réactionnel suivant :

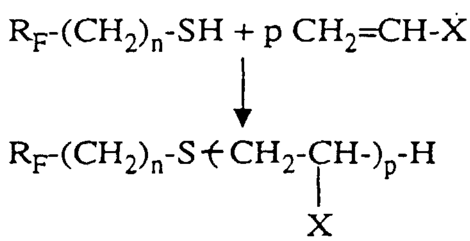

$R_F$, n, p et X ayant les mêmes significations que précédemment.

Des molécules de formule $CH_2$=CH-X sont notamment décrites dans les demandes de brevet français 2.197.023,

2.237.912, 2.596.400, 2.597.336, 2.617.399.

La réaction radicalaire a lieu en milieu solvant en présence d'un initiateur de radicaux libres.

Comme initiateurs de radicaux libres, on peut citer les hydroperoxydes tels que l'hydroperoxyde de tertiobutyle, les peroxydes tels que le peroxyde de dibenzoyle, les peresters tels que le peroxybenzoate de tertiobutyle ou les dérivés azoïques et en particulier l'azobisisobutyronitrile.

Les solvants utilisables doivent être inertes vis-à-vis des réactifs et peuvent être choisis parmi les alcools en $C_1$-$C_4$ tels que le méthanol, l'isopropanol, les alkyléthers, les éthers de glycol, les éthers cycliques tels que le tétrahydrofurane, les hydrocarbures aliphatiques ou aromatiques en $C_6$-$C_8$ comme le toluène.

Le mercaptan de départ est dissous dans le solvant en présence du composé filtrant comportant la double liaison réactive, puis l'initiateur de radicaux est additionné; la réaction étant effectuée sous atmosphère inerte.

Les composés de formule (I) pour lesquels p=1 peuvent être préparés également selon le schéma mentionné ci-dessus, en présence d'un catalyseur basique au lieu d'un initiateur de radicaux libres.

Comme catalyseurs basiques, on peut citer les métaux alcalins, les hydroxydes de métaux alcalins ou alcalino-terreux, les hydroxydes d'ammonium quaternaire tels que l'hydroxyde de benzyl triméthyl ammonium, les alcoolates des métaux alcalins tels que les méthylates ou tertiobutylates, les hydrures alcalins tels que l'hydrure de sodium, les amines tertiaires telles que la pyridine ou la triéthylamine. Ils peuvent aussi être des bases de Lewis parmi lesquelles on peut citer les fluorures de césium, de rubidium, de potassium. Ces composés peuvent être également supportés sur un solide tel que l'alumine. De préférence, on utilise un alcoolate alcalin comme le méthylate de sodium ou une amine tertiaire comme la pyridine.

De par leur caractère liposoluble, les composés de formule (I) ci-dessus se répartissent uniformément dans les supports cosmétiques classiques contenant au moins une phase grasse ou un solvant organique cosmétiquement acceptable et peuvent être appliqués sur la peau ou les cheveux pour constituer un film protecteur efficace.

La présente invention a donc aussi pour objet une composition cosmétique comprenant, dans un support cosmétiquement acceptable contenant au moins une phase grasse ou un solvant organique, une quantité efficace d'au moins un composé de formule (I) ci-dessus.

Le composé (I) est généralement présent dans des proportions comprises entre 0,1 et 15% en poids, par rapport au poids total de la composition.

La composition cosmétique de l'invention peut être utilisée comme composition protectrice de l'épiderme humain ou des cheveux contre les rayons ultraviolets, comme composition antisolaire ou comme produit de maquillage.

Cette composition peut se présenter en particulier sous forme de lotion, de lotion épaissie, de gel, de crème, de lait, de poudre, de bâtonnet solide et éventuellement être conditionnée en aérosol et se présenter sous forme de mousse ou de spray.

Elle peut contenir les adjuvants cosmétiques habituellement utilisés tels que des corps gras, des solvants organiques, des silicones, des épaississants, des adoucissants, des filtres solaires UV-A, UV-B ou à bande large, des agents anti-mousses, des agents hydratants, des parfums, des conservateurs, des tensio-actifs, des charges, des séquestrants, des polymères anioniques, cationiques, non-ioniques, amphotères ou leurs mélanges, des propulseurs, des agents alcalinisants ou acidifiants, des colorants, des pigments, ou tout autre ingrédient habituellement utilisé en cosmétique.

Parmi les solvants organiques, on peut citer les alcools et polyols inférieurs tels que l'éthanol, l'isopropanol, le propylèneglycol, la glycérine et le sorbitol.

Les corps gras peuvent être constitués par une huile ou une cire ou leur mélange, des acides gras, des esters d'acides gras, des alcools gras, la vaseline, la paraffine, la lanoline, la lanoline hydrogénée, la lanoline acétylée.

Les huiles sont choisies parmi les huiles animales, végétales, minérales ou de synthèse, et notamment l'huile de palme hydrogénée, l'huile de ricin hydrogénée, l'huile de vaseline, l'huile de paraffine, l'huile de Purcellin, les huiles de silicone et les isoparaffines.

Des huiles particulièrement préférées sont les huiles fluorées telles que celles décrites dans la demande WO 93/11103, et les perfluoropolyéthers vendus sous la dénomination de "FOMBLIN" par la Société MONTEFLUO.

Lorsque la composition cosmétique selon l'invention est utilisée pour la protection de l'épiderme humain contre les rayons UV ou comme composition antisolaire, elle peut se présenter sous forme de suspension ou de dispersion dans des solvants ou des corps gras, ou encore sous forme d'émulsion telle qu'une crème ou un lait, de dispersion vésiculaire, sous forme de pommade, de gel, de bâtonnet solide ou de mousse aérosol. Les émulsions peuvent contenir en outre des agents tensio-actifs anioniques, non-ioniques, cationiques ou amphotères.

Lorsque la composition cosmétique selon l'invention est utilisée pour la protection des cheveux, elle peut se présenter sous forme de shampooing, de lotion, de gel ou composition à rincer, à appliquer avant ou après shampooing, avant ou après coloration ou décoloration, avant, pendant ou après permanente ou défrisage, de lotion ou gel coiffants ou traitants, de lotion ou gel pour le brushing ou la mise en plis, de laque pour cheveux, de composition de permanente ou de défrisage, de coloration ou décoloration des cheveux.

Lorsque la composition est utilisée comme produit de maquillage des cils, des sourcils, de la peau, des ongles ou

des cheveux, tel que crème de traitement de l'épiderme, fond de teint, bâton de rouge à lèvres, fard à paupières, fard à joues, ligneur, mascara, gel colorant, elle peut se présenter sous forme solide ou pâteuse, anhydre ou aqueuse, comme des émulsions huile-dans-eau ou eau-dans-huile, des suspensions ou encore des gels.

L'invention a aussi pour objet un procédé de protection de l'épiderme humain et des cheveux contre le rayonnement ultraviolet, consistant à appliquer sur la peau ou les cheveux une quantité efficace de la composition cosmétique ci-dessus.

Les exemples qui suivent illustrent l'invention sans toutefois revêtir un caractère limitatif.

**EXEMPLES DE PREPARATION**

EXEMPLE 1

Préparation du composé de formule :

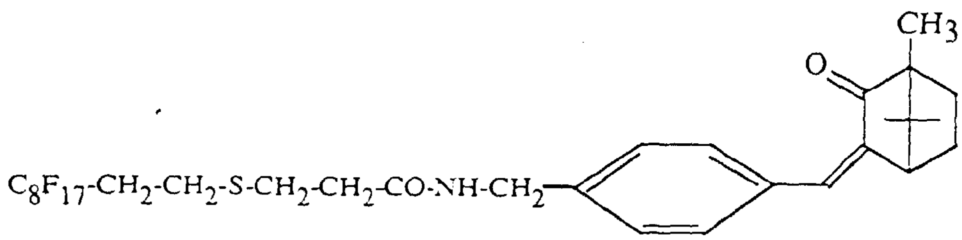

Dans un réacteur, on solubilise 40 g (0,123 mole) de [3-(4'-acrylamidométhyl benzylidène)] D,L-camphre (composé décrit à l'exemple 5 de la demande française FR 2.237.912) dans 65 ml de méthanol à 35°C, sous courant d'azote.

Lorsque la température de la solution est redescendue à 25-30°C, on ajoute en 5 minutes 59,25 g (0,123 mole) de 2-F-octyl éthane thiol (produit commercialisé par la Société ATOCHEM sous la dénomination de "FORALKYL EM8").

On chauffe à nouveau et lorsque la température du mélange atteint 50°C, on ajoute 1,05 g (matière active) d'hydroxyde de benzyl triméthyl ammonium (vendu à 35% en solution méthanolique sous la dénomination "TRITON B" par la Société FLUKA), en 5 minutes environ.

Après 4 heures de chauffage au reflux du méthanol, le solvant est évaporé sous pression réduite. Le résidu solide ainsi obtenu est repris par 200 ml d'hexane, puis filtré. Après un second empâtage à l'hexane suivi d'une filtration, le résidu est successivement lavé : deux fois par 150 ml d'eau à 40°C, puis deux fois par 150 ml d'éthanol à 25°C.

Le produit est enfin recristallisé dans l'éthanol.

On obtient 75 g (rendement 76%) d'un solide blanc dont le point de fusion est de 100°C.

La pureté du produit est contrôlée par chromatographie sur couche mince (plaques silice Merck Kieselgel F 254, éluant dichlorométhane/méthanol : 39/1).

| ANALYSE ELEMENTAIRE : | | | | | |
|---|---|---|---|---|---|
| | C | H | N | S | F |
| Calculé | 46,33 | 3,76 | 1,74 | 3,99 | 40,19 |
| Trouvé | 46,33 | 3,81 | 1,77 | 3,88 | 40,25 |

$a_s$ 297 nm = 33

$a_s$ est l'absorbance spécifique définie par la Norme Française T. 01030 (janvier 1972).

Précédemment, la puissance absorbante du filtre était exprimée à l'aide de la valeur de Ksp (K spécifique), qui est une fonction de la quantité de substance filtrante contenue dans l'échantillon, de la densité optique mesurée et d'une constante dépendant de l'appareil.

La définition de Ksp est donnée dans l'ouvrage "Introduction to electronic absorption Spectroscopy in organic chemistry" par Gillian and Stern, Arnold Ed., London 1954, page 10.

$$Ksp = \frac{K}{c}$$

avec

$$K = \frac{d}{1}$$

d = densité optique mesurée
c = concentration de la solution (g/ml)
1= épaisseur de la cellule en cm

Actuellement, la puissance absorbante est définie par le terme "absorbance spécifique, $a_s$", reliée au Ksp par la relation

$$\frac{Ksp}{a_s} = 1000.$$

EXEMPLE 2

Préparation du composé de formule :

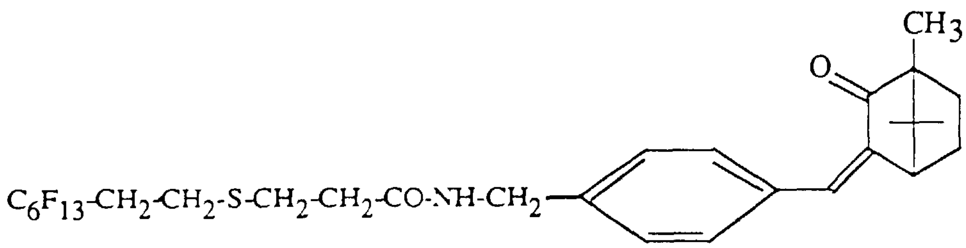

Le composé est préparé selon le mode opératoire de l'exemple 1, en utilisant :

- 10 g (0,03 mole) de [3-(4'-acrylamidométhylbenzylidène)] D,L-camphre solubilisés dans 20 ml de méthanol;
- 11,7 g (0,03 mole) de 2-F-hexyl éthane thiol;
- 0,26 g d'hydroxyde de benzyl triméthyl ammonium.

A la fin de la réaction, le milieu réactionnel décante.

La phase inférieure est récupérée et lavée à l'hexane. Le précipité obtenu, filtré, est lavé par 50 ml d'une solution eau/éthanol 80/20 (vol/vol), puis par 100 ml d'eau.

Après filtration et séchage, on obtient 16,3 g (75%) d'une poudre blanche dont le point de fusion est de 92°C. La pureté du produit obtenu est contrôlée par chromatographie sur couche mince (plaques silice Merck Kieselgel F 254, éluant dichlorométhane/méthanol 39/1).

| ANALYSE ELEMENTAIRE : | | | | | |
|---|---|---|---|---|---|
| | C | H | N | S | F |
| Calculé | 49,50 | 4,30 | 1,99 | 4,56 | 35,10 |
| Trouvé | 49,67 | 4,25 | 1,95 | 4,31 | 34,84 |

$a_s$ 297 nm = 36

EXEMPLE 3

Préparation du composé de formule (I) pour lequel $R_F = C_8F_{17}$ - n = 2, p = 3, et

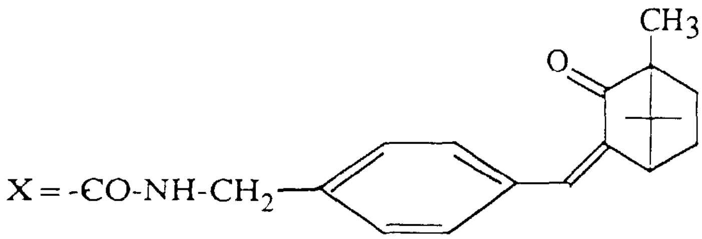

Dans un réacteur, on solubilise 30 g (0,09 mole) de [3-(4'-acrylamidométhyl benzylidène)] D,L-camphre dans 50 ml de méthanol, à 35°C, sous courant d'azote. Lorsque la température de la solution est redescendue à 25-30°C, on ajoute 14,9 g (0,031 mole) de 2-F-octyl éthane thiol. On chauffe à nouveau et, lorsque la température du mélange atteint 50°C, on ajoute en 30 minutes une solution de 1,2 g d'azobisisobutyronitrile dans 50 ml de méthanol, tout en poursuivant la montée en température jusqu'au reflux du méthanol.

Après 14 heures de chauffage, on ajoute 0,175 g d'hydroxyde de benzyl triméthyl ammonium et on maintient le reflux pendant 2 heures.

Le méthanol est ensuite évaporé, le résidu repris avec 200 ml d'éther éthylique, puis le produit est amené à sec sous pression réduite. On obtient ainsi 46 g d'une poudre orangée. Ce solide est alors lavé sous agitation par 200 ml d'heptane pendant 10 minutes, le précipité étant isolé par filtration puis séché sous vide à l'étuve. On isole ainsi 45 g d'une poudre jaune dont le point de fusion est de 81°C.

Indice de thioéther : 0,66 meq/g (théorique : 0,67 meq/g)

| ANALYSE ELEMENTAIRE : | | | | | |
|---|---|---|---|---|---|
| | C | H | N | S | F |
| Calculé | 60,45 | 5,56 | 2,90 | 2,21 | 22,27 |
| Trouvé | 60,41 | 5,60 | 2,96 | 2,07 | 21,99 |

$a_s$ 297 nm = 47

EXEMPLE 4

Préparation du composé de formule :

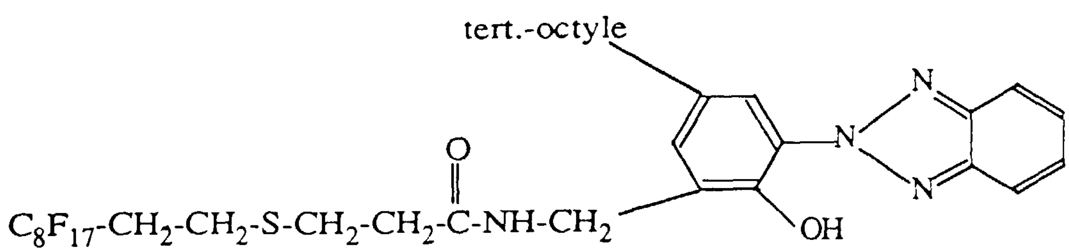

Dans un réacteur, on solubilise 122 g de [2-(2'-hydroxy 3'-acrylamidométhyl 5'-tert.-octyl phényl)] 2H-benzotriazole (composé décrit dans l'exemple 3, stade 1, de la demande française 2.597.336) dans 800 ml de méthanol, en chauffant le mélange à 50°C, sous courant d'azote. On ajoute 151,2 g (0,315 mole) de 2-F-octyléthanethiol en 20 minutes en maintenant la température à 50°C. On ajoute ensuite en 10 minutes, 2,5 g d'hydroxyde de benzyl triméthyl ammonium.

Le mélange est chauffé au reflux du méthanol pendant 6 heures. Un précipité beige se forme. On ajoute environ 700 ml de méthanol porté à la température de 50°C. On agite puis on filtre. On effectue un empâtage avec 400 ml de méthanol, puis avec 400 ml d'éthanol. On obtient 160 g (62%) d'une poudre beige dont le point de fusion est de 155°C. La pureté du produit est contrôlée par chromatographie sur couche mince (plaques silice Merck Kieselgel F254, éluant : dichlorométhane/méthanol : 39/1).

| ANALYSE ELEMENTAIRE : | | | | | |
|---|---|---|---|---|---|
| | C | H | N | S | F |
| Calculé | 46,05 | 3,98 | 6,32 | 3,62 | 36,42 |
| Trouvé | 46,04 | 4,02 | 6,27 | 3,75 | 36,60 |

à 305 nm : $a_{s1}$ = 19,2
à 340 nm : $a_{s2}$ = 20,5.

EXEMPLE 5

Préparation du composé de formule :

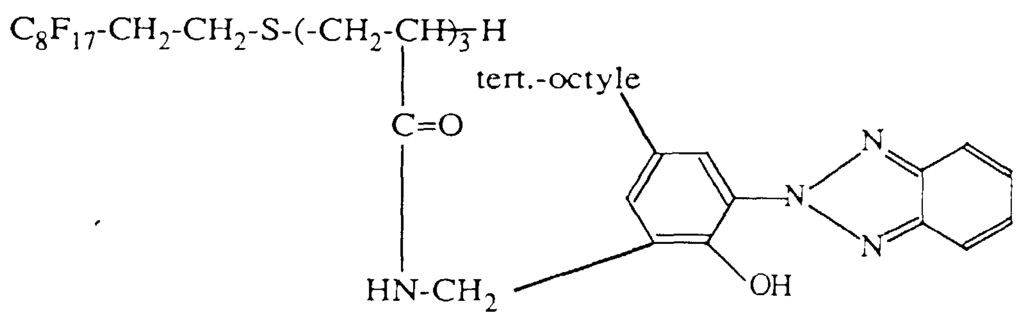

Dans un réacteur, on solubilise 183 g (0,45 mole) de [2-(2'-hydroxy-3'-acrylamidométhyl-5'-tert.-octylphényl)] 2H-benzotriazole dans 250 ml de tétrahydrofuranne, sous courant d'azote, en portant la température à 60°C en 30 minutes. On ramène la température de la solution obtenue à 40°C et on additionne 72 g (0,15 mole) de 2-F-octyléthanethiol en 15 minutes, puis on porte la température à 70°C et on additionne en 30 minutes une solution de 5,5 g d'azobisisobutyronitrile dans 150 ml de tétrahydrofuranne.

Après 16 heures de chauffage, on ajoute 0,5 g d'hydroxyde de benzyl triméthyl ammonium et on poursuit le chauffage à 70°C pendant 2 heures. Après retour à 25°C, le produit est alors précipité goutte-à-goutte dans 4 l d'heptane en 20 minutes. Après filtration, on obtient 220 g (85%) d'une poudre beige dont le point de fusion est de 82°C.

Indice de thioéther : 0,58 meq/g (théorique : 0,58 meq/g).

| ANALYSE ELEMENTAIRE : | | | | | |
|---|---|---|---|---|---|
| | C | H | N | S | F |
| Calculé | 58,04 | 5,47 | 9,91 | 1,89 | 19,04 |
| Trouvé | 58,17 | 5,50 | 9,93 | 1,87 | 18,73 |

à 305 nm : $a_{s1}$ = 27
à 340 nm : $a_{s2}$ = 28.

**EXEMPLES DE FORMULATION**

EXEMPLE 1

**Crème solaire pour la peau - Emulsion huile-dans-eau**

- Mélange (80/20) d'alcool cétylstéarylique et d'alcool cétylstéarylique oxyéthyléné à 33 moles d'oxyde d'éthylène, vendu sous la dénomination "SINNOWAX AO" par la Société HENKEL 7 g
- Mélange de mono- et distéarate de glycérol non autoémulsionnable 2 g
- Alcool cétylique 1,5 g
- Polydiméthylsiloxane vendu sous la dénomination "SILICONE DC 200-350" par la Société DOW CORNING

1,5 g

- 1-(2-F-hexyléthylthio)3-(2"-éthylhexyloxy)-2-propanol 20 g
- Composé de l'exemple 4 1,4 g
- Glycérine 15 g
- Conservateurs qs
- Eau qsp 100 g

On réalise l'émulsion en additionnant la phase grasse contenant le filtre, portée aux environs de 80°C à la phase aqueuse renfermant les conservateurs et la glycérine à la même température et sous agitation rapide.

<u>EXEMPLE 2</u>

**Lait solaire - Emulsion eau-dans-huile**

- Hydroxystéarate de sorbitan et de glycérol oxyéthyléné à 2,5 moles d'oxyde d'éthylène et oxypropyléné à 1,5 moles d'oxyde de propylène, vendu sous la dénomination "ARLACEL 780" par la Société ICI 5 g
- Huile de vaseline 14 g
- 12-hydroxystéarate de β-hydroxyoctacosanyle vendu sous la dénomination "ELFACOS C 26" par la Société AKZO 6 g
- 1-(2'-F-octyléthylthio) 3-(2"-éthylhexyloxy)-2 propanol 10 g
- Composé de l'exemple 3 1 g
- Glycérine 4 g
- Conservateurs qs
- Eau qsp 100 g

On réalise cette émulsion en additionnant la phase aqueuse renfermant les conservateurs et la glycérine portée aux environs de 80°C à la phase grasse portée à la même température sous agitation vive.

## Revendications

**1.** Nouveaux composés répondant à la formule (I) :

$$R_F\text{-}(CH_2)_n\text{-}S\text{-}(CH_2\text{-}\underset{\displaystyle X}{\underset{|}{C}}H\text{-})_p\text{ - }H \qquad (I)$$

dans laquelle :

$R_F$ est un radical perfluoroalkyle, linéaire ou ramifié, en $C_4$-$C_{10}$, de préférence en $C_6$-$C_8$, ou un mélange de radicaux perfluoroalkyle, linéaires ou ramifiés, en $C_4$-$C_{10}$, de préférence en $C_6$-$C_8$;
n est un nombre entier compris entre 0 et 4, de préférence égal à 2;
p est un nombre entier ou statistique compris entre 1 et 10, de préférence compris entre 1 et 5; et
X est un groupement aromatique conférant au composé de formule (I) une absorption de l'ultraviolet dans la zone de longueurs d'onde comprise entre 280 à 400 nm.

**2.** Composés selon la revendication 1, caractérisés en ce que dans la formule (I), X représente le groupement

$$\text{-}\underset{\displaystyle O}{\underset{\|}{C}}\text{-NH-}CH_2\text{-}Y,$$

avec Y représentant un radical aromatique absorbant le rayonnement ultraviolet dans le domaine de longueurs d'onde allant de 280 à 400 nm.

**3.** Composés selon la revendication 2, caractérisés en ce que Y est choisi parmi les groupements :

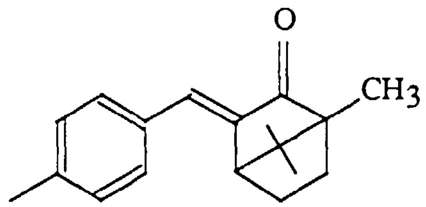

(II)

et

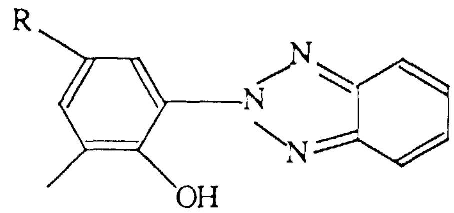

(III)

R désignant le radical méthyle ou tert.-octyle.

**4.** Procédé de préparation des composés nouveaux répondant à la formule (I) :

$$R_F\text{-}(CH_2)_n\text{-}S\text{-}(CH_2\text{-}\underset{\displaystyle X}{\underset{|}{C}}H\text{-})_p\text{ - }H \qquad \text{(I)}$$

dans laquelle :

> $R_F$ est un radical perfluoroalkyle, linéaire ou ramifié, en $C_4$-$C_{10}$, de préférence en $C_6$-$C_8$, ou un mélange de radicaux perfluoroalkyle, linéaires ou ramifiés, en $C_4$-$C_{10}$, de préférence en $C_6$-$C_8$;
> n est un nombre entier compris entre 0 et 4, de préférence égal à 2;
> p est un nombre entier ou statistique compris entre 1 et 10, de préférence compris entre 1 et 5; et
> X est un groupement aromatique conférant au composé de formule (I) une absorption de l'ultraviolet dans la zone de longueurs d'onde comprise entre 280 à 400 nm,

caractérisé en ce qu'il consiste en une addition radicalaire en milieu solvant d'un mercaptan comportant une chaîne perfluoroalkyle sur une ou plusieurs molécules absorbant le rayonnement ultraviolet dans le domaine de longueurs d'onde allant de 280 à 400 nm et comportant un groupe contenant une double liaison réactive ne participant pas à l'absorption UV selon le schéma réactionnel suivant :

$$R_F\text{-}(CH_2)_n\text{-}SH + p\ CH_2{=}CH\text{-}X$$
$$\downarrow$$
$$R_F\text{-}(CH_2)_n\text{-}S\text{-}(CH_2\text{-}\underset{\displaystyle X}{\underset{|}{C}}H\text{-})_p\text{-}H$$

avec $R_F$, n, p et X ayant la même définition que dans la formule (I), sous atmosphère inerte en présence d'un initiateur de radicaux libres.

**5.** Procédé de préparation des composés nouveaux répondant à la formule :

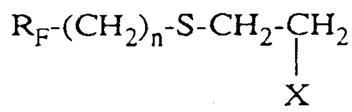

dans laquelle :

$R_F$ est un radical perfluoroalkyle, linéaire ou ramifié, en $C_4$-$C_{10}$, de préférence en $C_6$-$C_8$, ou un mélange de radicaux perfluoroalkyle, linéaires ou ramifiés, en $C_4$-$C_{10}$, de préférence en $C_6$-$C_8$;
n est un nombre entier compris entre 0 et 4, de préférence égal à 2;
X est un groupement aromatique conférant au composé de formule (I) une absorption de l'ultraviolet dans la zone de longueurs d'onde comprise entre 280 à 400 nm,

caractérisé en ce qu'il consiste en une addition radicalaire en milieu solvant d'un mercaptan comportant une chaîne perfluoro sur une ou plusieurs molécules absorbant le rayonnement ultraviolet dans le domaine de longueurs d'onde choisi et comportant un groupe contenant une double liaison réactive selon le schéma réactionnel suivant :

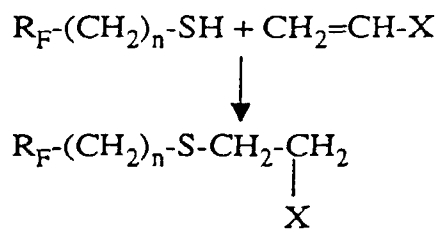

avec $R_F$, n et X ayant la même signification que précédemment, sous atmosphère inerte en présence d'un catalyseur basique.

**6.** Utilisation des composés de formule (I) :

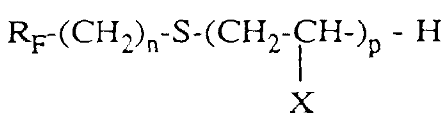
(I)

dans laquelle :

$R_F$ est un radical perfluoroalkyle, linéaire ou ramifié, en $C_4$-$C_{10}$, de préférence en $C_6$-$C_8$, ou un mélange de radicaux perfluoroalkyle, linéaires ou ramifiés, en $C_4$-$C_{10}$, de préférence en $C_6$-$C_8$;
n est un nombre entier compris entre 0 et 4, de préférence égal à 2;
p est un nombre entier ou statistique compris entre 1 et 10, de préférence compris entre 1 et 5; et
X est un groupement aromatique conférant au composé de formule (I) une absorption de l'ultraviolet dans la zone de longueurs d'onde comprise entre 280 à 400 nm;

à titre de filtres solaires dans le domaine des UV-A et UV-B.

**7.** Composition cosmétique filtrant les rayons ultraviolets, caractérisée en ce qu'elle contient, dans un support cosmétiquement acceptable, une quantité efficace d'au moins un filtre solaire à chaîne perfluorocarbonée de formule (I) :

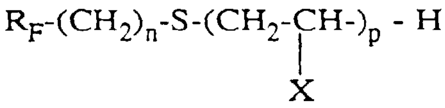

(I)

dans laquelle :

$R_F$ est un radical perfluoroalkyle, linéaire ou ramifié, en $C_4$-$C_{10}$, de préférence en $C_6$-$C_8$, ou un mélange de radicaux perfluoroalkyle, linéaires ou ramifiés, en $C_4$-$C_{10}$, de préférence en $C_6$-$C_8$;
n est un nombre entier compris entre 0 et 4, de préférence égal à 2;
p est un nombre entier ou statistique compris entre 1 et 10, de préférence compris entre 1 et 5; et
X est un groupement aromatique conférant au composé de formule (I) une absorption de l'ultraviolet dans la zone de longueurs d'onde comprise entre 280 à 400 nm.

**8.** Composition cosmétique selon la revendication 7, caractérisée en ce que dans la formule (I), X désigne le groupement

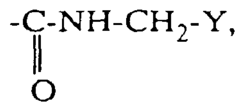

Y représentant un radical aromatique absorbant le rayonnement ultraviolet dans le domaine de longueurs d'onde allant de 280 à 400 nm.

**9.** Composition cosmétique selon la revendication 8, caractérisée en ce que Y est choisi parmi :

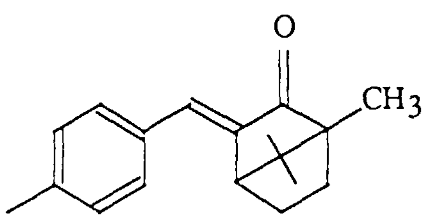

(II)

et

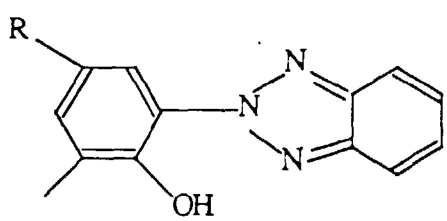

(III)

R désignant le radical méthyle ou tert.-octyle.

**10.** Composition cosmétique selon l'une quelconque des revendications 7 à 9, caractérisée en ce que le support cosmétiquement acceptable contient au moins une phase grasse ou un solvant organique.

**11.** Composition cosmétique selon l'une quelconque des revendications 7 à 10, caractérisée en ce que le composé de formule (I) est présent dans la composition dans des proportions comprises entre 0,1 et 15% en poids par

rapport au poids total de la composition.

**12.** Composition cosmétique selon l'une quelconque des revendications 7 à 11, caractérisée en ce qu'elle se présente sous forme de lotion, de gel, de crème, de lait, de poudre, de bâtonnet solide et est éventuellement conditionnée en aérosol et se présente sous forme de mousse ou de spray.

**13.** Composition cosmétique selon l'une quelconque des revendications 7 à 12, caractérisée en ce qu'elle contient en outre au moins un adjuvant choisi parmi les corps gras, les solvants organiques, les silicones, les épaississants, les adoucissants, les filtres solaires UV-A, UV-B ou à bande large, les agents antimousses, les agents hydratants, les parfums, les conservateurs, les tensio-actifs, les charges, les séquestrants, les polymères anioniques, cationiques, nonioniques, amphotères ou leurs mélanges, les propulseurs, les agents alcalinisants ou acidifiants, les colorants, les pigments.

**14.** Composition cosmétique selon l'une quelconque des revendications 7 à 13, constituant une composition protectrice de l'épiderme humain contre les rayons ultraviolets ou antisolaire, caractérisée par le fait qu'elle se présente sous forme de suspension ou de dispersion dans des solvants ou corps gras, d'émulsion, de dispersion vésiculaire, de pommade, de gel, de bâtonnet solide ou de mousse aérosol.

**15.** Composition cosmétique selon l'une quelconque des revendications 7 à 13, pour la protection des cheveux contre les rayons ultraviolets, caractérisée par le fait qu'elle se présente sous forme de shampooing, de lotion, de gel ou composition à rincer, à appliquer avant ou après shampooing, avant ou après coloration ou décoloration, avant, pendant ou après permanente ou défrisage, sous forme de lotion ou de gel coiffants ou traitants, de lotion ou gel pour le brushing ou la mise en plis, de laque pour cheveux, de composition de permanente ou de défrisage, de coloration ou de décoloration des cheveux.

**16.** Composition cosmétique selon l'une quelconque des revendications 7 à 13, constituant un produit de maquillage des cils, des sourcils, de la peau, des ongles ou des cheveux, caractérisée par le fait qu'elle se présente sous forme de crème de traitement de l'épiderme, de fond de teint, de bâton de rouge à lèvres, de fard à paupières, de fard à joues, de ligneur, de mascara, de gel colorant et se présente sous forme solide ou pâteuse, anhydre ou aqueuse.

**17.** Procédé de protection de l'épiderme humain et des cheveux contre le rayonnement ultraviolet, caractérisé par le fait qu'il consiste à appliquer sur la peau ou les cheveux une quantité efficace d'un filtre solaire à chaîne perfluorocarbonée tel que défini dans l'une des revendications 1 à 3.

## Patentansprüche

**1.** Neue Verbindungen der Formel (I) :

$$R_F\text{-}(CH_2)_n\text{-}S\text{-}(CH_2\text{-}\underset{\underset{X}{|}}{C}H\text{-})_p - H \qquad (I)$$

worin gilt:

$R_F$ ist ein linearer oder verzweigter $C_{4-10}$- und vorzugsweise ein $C_{6-8}$-Perfluoralkylrest oder eine Mischung dieser Reste;
n ist eine ganze Zahl von 0 bis 4 und vorzugsweise gleich 2;
p ist eine ganze oder statistische Zahl von 1 bis 10 und vorzugsweise von 1 bis 5; und
X ist eine aromatische Gruppierung, die der Verbindung der Formel (I) eine Absorption der ultravioletten Strahlung im Wellenlängenbereich von 280 bis 400 nm verleiht.

**2.** Verbindungen gemäß Anspruch 1,
dadurch **gekennzeichnet**, daß

in der Formel (I) X die Gruppierung darstellt:

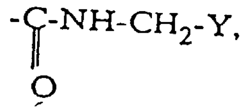

worin Y einen aromatischen Rest darstellt, der die ultraviolette Strahlung im Wellenlängenbereich von 280 bis 400 nm absorbiert.

**3.** Verbindungen gemäß Anspruch 2,
dadurch **gekennzeichnet**, daß
Y aus den Gruppierungen ausgewählt ist:

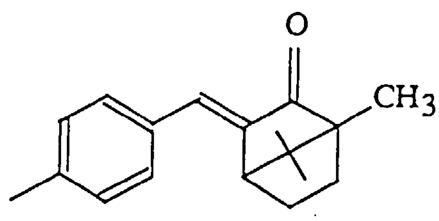

(II)

und

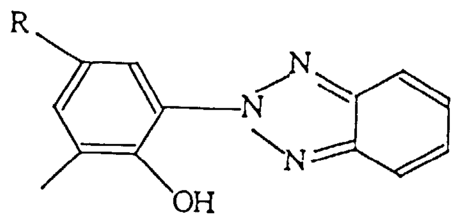

(III)

worin R den Methyl- oder t-Octylrest bedeutet.

**4.** Verfahren zur Herstellung der neuen Verbindungen der Formel (I) :

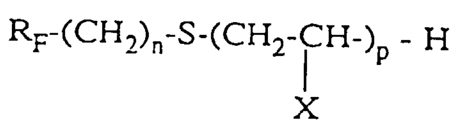

(I)

worin gilt:

$R_F$ ist ein linearer oder verzweigter $C_{4-10}$- und vorzugsweise ein $C_{6-8}$-Perfluoralkylrest oder eine Mischung dieser Reste;
n ist eine ganze Zahl von 0 bis 4 und vorzugsweise gleich 2;
p ist eine ganze oder statistische Zahl von 1 bis 10 und vorzugsweise von 1 bis 5; und
X ist eine aromatische Gruppierung, die der Verbindung der Formel (I) eine Absorption der ultravioletten Strahlung im Wellenlängenbereich von 280 bis 400 nm verleiht,

dadurch **gekennzeichnet**, daß

es auf einer in einem Lösungsmittelmilieu durchgeführten radikalischen Addition eines Merkaptans mit einer Perfluoralkylkette an ein oder mehrere Moleküle, die die ultraviolette Strahlung im Wellenlängenbereich von 280 bis 400 nm absorbieren und eine Gruppe mit einer reaktiven Doppelbindung, die nicht an der UV-Absorption teilnimmt, enthalten, gemäß dem folgenden Reaktionsschema beruht:

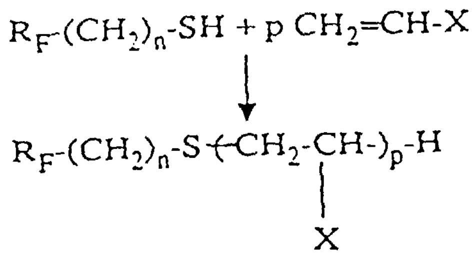

worin $R_F$, n, p und X dieselbe Definition wie in Formel (I) haben,
wobei die Reaktion unter Inertatmosphäre in Gegenwart eines Initiators für freie Radikale durchgeführt wird.

**5.** Verfahren zur Herstellung der neuen Verbindungen der Formel:

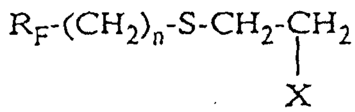

worin gilt:

$R_F$ ist ein linearer oder verzweigter $C_{4-10}$- und vorzugsweise ein $C_{6-8}$-Perfluoralkylrest oder eine Mischung dieser Reste;
n ist eine ganze Zahl von 0 bis 4 und vorzugsweise gleich 2;
X ist eine aromatische Gruppierung, die der Verbindung der Formel eine Absorption der ultravioletten Strahlung im Wellenlängenbereich von 280 bis 400 nm verleiht,

dadurch **gekennzeichnet**, daß

es auf einer in einem Lösungsmittelmilieu durchgeführten radikalischen Addition eines Merkaptans mit einer Perfluorkette an ein oder mehrere Moleküle, die die ultraviolette Strahlung im ausgewählten Wellenlängenbereich absorbieren und eine Gruppe mit einer reaktiven Doppelbindung enthalten, gemäß dem folgenden Reaktionsschema beruht:

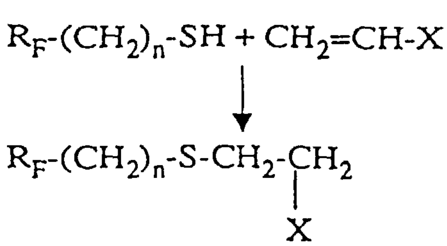

worin $R_F$, n und X die vorstehend angegebene Bedeutung haben,
wobei die Reaktion unter Inertatmosphäre in Gegenwart eines basischen Katalysators durchgeführt wird.

**6.** Verwendung der Verbindungen der Formel (I) :

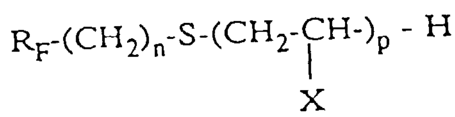

(I)

worin gilt:

$R_F$ ist ein linearer oder verzweigter $C_{4-10}$- und vorzugsweise ein $C_{6-8}$-Perfluoralkylrest oder eine Mischung dieser Reste;
n ist eine ganze Zahl von 0 bis 4 und vorzugsweise gleich 2;
p ist eine ganze oder statistische Zahl von 1 bis 10 und vorzugsweise von 1 bis 5; und
X ist eine aromatische Gruppierung, die der Verbindung der Formel (I) eine Absorption der ultravioletten Strahlung im Wellenlängenbereich von 280 bis 400 nm verleiht, als Sonnenfilterstoffe im Bereich der UV-A- und UV-B-Strahlen.

**7.** Kosmetische Zusammensetzung, die die ultravioletten Strahlen filtert,
dadurch **gekennzeichnet**, daß
sie, in einem kosmetisch geeigneten Trägermedium, eine wirksame Menge mindestens eines Sonnenfilterstoffs mit Perfluorkohlenstoffkette der Formel (I) enthält:

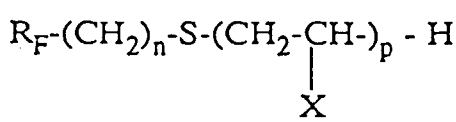

(I)

worin gilt:

$R_F$ ist ein linearer oder verzweigter $C_{4-10}$- und vorzugsweise ein $C_{6-8}$-Perfluoralkylrest oder eine Mischung dieser Reste;
n ist eine ganze Zahl von 0 bis 4 und vorzugsweise gleich 2;
p ist eine ganze oder statistische Zahl von 1 bis 10 und vorzugsweise von 1 bis 5; und
X ist eine aromatische Gruppierung, die der Verbindung der Formel (I) eine Absorption der ultravioletten Strahlung im Wellenlängenbereich von 280 bis 400 nm verleiht.

**8.** Kosmetische Zusammensetzung gemäß Anspruch 7,
dadurch **gekennzeichnet**, daß
in der Formel (I) X die Gruppierung bedeutet:

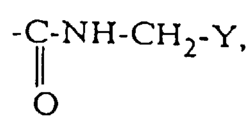

worin Y einen aromatischen Rest darstellt, der die ultraviolette Strahlung im Wellenlängenbereich von 280 bis 400 nm absorbiert.

**9.** Kosmetische Zusammensetzung gemäß Anspruch 8,
dadurch **gekennzeichnet**, daß
Y ausgewählt ist aus:

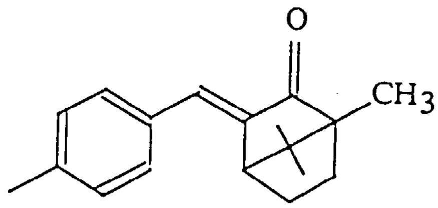

(II)

und

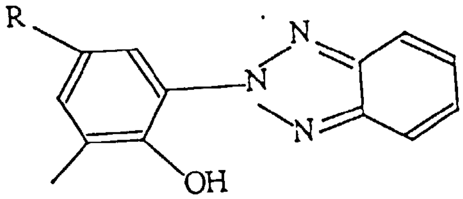

(III)

worin R den Methyl- oder t-Octylrest bedeutet.

**10.** Kosmetische Zusammensetzung gemäß einem der Ansprüche 7 bis 9,
dadurch **gekennzeichnet**, daß
das kosmetisch geeignete Trägermedium mindestens eine Fettphase und ein organisches Lösungsmittel enthält.

**11.** Kosmetische Zusammensetzung gemäß einem der Ansprüche 7 bis 10,
dadurch **gekennzeichnet**, daß
die Verbindung der Formel (I) in der Zusammensetzung in Mengenanteilen von 0,1 bis 15 Gew.% vorhanden ist, bezogen auf das Gesamtgewicht der Zusammensetzung.

**12.** Kosmetische Zusammensetzung gemäß einem der Ansprüche 7 bis 11,
dadurch **gekennzeichnet**, daß
sie in Form einer Lotion, eines Gels, einer Creme, einer Milch, eines Pulvers oder eines Feststoffstäbchens vorliegt und gegebenenfalls als Aerosol zubereitet ist und in Form eines Schaums oder Spray vorliegt.

**13.** Kosmetische Zusammensetzung gemäß einem der Ansprüche 7 bis 12,
dadurch **gekennzeichnet**, daß
sie ausserdem mindestens einen Hilfsstoff enthält, ausgewählt aus Fettkörpern, organischen Lösungsmitteln, Siliconen, Verdickungsmitteln, Weichmachern, Sonnenfilterstoffen für UV-A, UV-B oder den langwelligen Bereich, aus Antischaummitteln, Hydratisiermitteln, Parfüm-Produkten, Konservierungsstoffen, oberflächenaktiven Mitteln, Beaufschlagungsmitteln, Sequestriermitteln, anionischen, kationischen, nichtionischen und amphoteren Polymeren oder aus deren Mischungen, aus Treibmitteln, alkalisch oder sauer stellenen Mitteln, Farbstoffen und aus Pigmenten.

**14.** Kosmetische Zusammensetzung gemäß einem der Ansprüche 7 bis 13,
welche eine Zusammensetzung zum Schutz der menschlichen Epidermis vor den ultravioletten Strahlen oder eine Sonnenschutzmittelzusammensetzung darstellt,
dadurch **gekennzeichnet**, daß
sie in Form einer Suspension oder Dispersion in Lösungsmitteln oder Fettkörpern, in Form einer Emulsion, bläschenartigen Dispersion, Pomade, eines Gels, eines Feststoffstäbchens oder eines Aerosol-Schaums vorliegt.

**15.** Kosmetische Zusammensetzung gemäß einem der Ansprüche 7 bis 13
zum Schutz der Haare vor den ultravioletten Strahlen,
dadurch **gekennzeichnet**, daß
sie in Form eines Shampoo, einer Lotion, eines Gels oder einer Zusammensetzung zur Spülung, zur Aufbringung vor oder nach dem Schamponieren, vor oder nach einer Färbung oder Entfärbung, vor, bei oder nach einer Dauerwelle oder einem Entfrisiervorgang, in Form einer Lotion oder eines Gels zum Frisieren oder Behandeln, einer Lotion oder eines Gels zum Ausbürsten oder zur Wellengebung, eines Haarlacks, einer Zusammensetzung zur Dauerwelle oder zum Entfrisieren, zur Färbung oder Entfärbung der Haare vorliegt.

**16.** Kosmetische Zusammensetzung gemäß einem der Ansprüche 7 bis 13,
welche ein Produkt zum Schminken der Wimpern, Augenbrauen, Haut, Nägel oder der Haare darstellt,
dadurch **gekennzeichnet**, daß
sie in Form einer Creme zum Behandeln der Epidermis, einer Teint-Grundlage, eines Lippenstifts, einer Schminke für Lidschatten, einer Wangenschminke, eines Liniermittels, einer Wimperntusche oder eines Färbegels vorliegt und in fester oder pastöser, wasserfreier oder wasserhaltiger Form zubereitet ist.

**17.** Verfahren zum Schutz der menschlichen Epidermis und der Haare vor der ultravioletten Strahlung,
dadurch **gekennzeichnet**, daß
es darauf beruht, daß man auf die Haut oder die Haare eine wirksame Menge eines in einem der Ansprüche 1 bis 3 definierten Sonnenfilterstoffs mit Perfluorkohlenstoffkette aufträgt.

## Claims

**1.** Novel compounds corresponding to formula (I) :

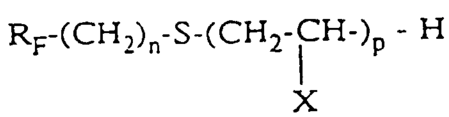

(I)

in which:

$R_F$ is a linear or branched $C_4$-$C_{10}$, preferably $C_6$-$C_8$, perfluoroalkyl radical or a mixture of linear or branched $C_4$-$C_{10}$, preferably $C_6$-$C_8$, perfluoroalkyl radicals;
n is an integer between 0 and 4, preferably equal to 2;
p is an integer or statistical number between 1 and 10, preferably between 1 and 5; and
X is an aromatic group which imparts to the compound of formula (I) absorption of ultraviolet in the wavelength region between 280 and 400 nm.

**2.** Compounds according to Claim 1, characterized in that, in formula (I), X represents the group

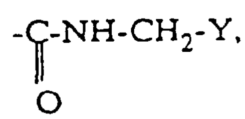

with Y representing an aromatic radical which absorbs ultraviolet radiation in the wavelength region ranging from 280 to 400 nm.

**3.** Compounds according to Claim 2, characterized in that Y is chosen from the groups:

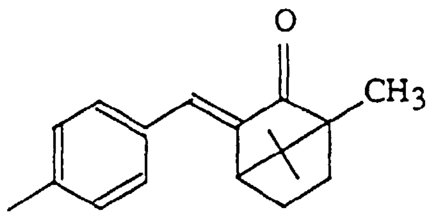

(II)

and

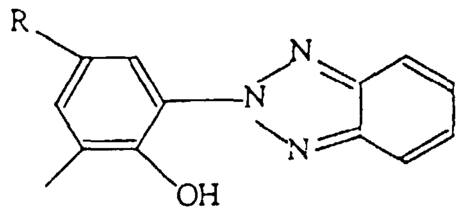

(III)

R denoting a methyl or tert-octyl radical.

**4.** Process for the preparation of the novel compounds corresponding to formula (I):

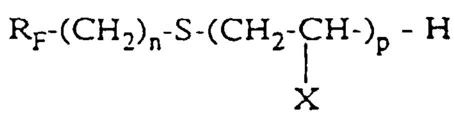

(I)

in which:

$R_F$ is a linear or branched $C_4$-$C_{10}$, preferably $C_6$-$C_8$, perfluoroalkyl radical or a mixture of linear or branched $C_4$-$C_{10}$, preferably $C_6$-$C_8$, perfluoroalkyl radicals;
n is an integer between 0 and 4, preferably equal to 2;
p is an integer or statistical number between 1 and 10, preferably between 1 and 5; and
X is an aromatic group which imparts to the compound of formula (I) absorption of ultraviolet in the wavelength region between 280 and 400 nm,

characterized in that it consists of a radical addition, in a solvent medium, of a mercaptan containing a perfluoroalkyl chain to one or more molecules which absorb ultraviolet radiation in the wavelength region ranging from 280 to 400 nm and which contain a group containing a reactive double bond that does not participate in the UV absorption, according to the following reaction scheme:

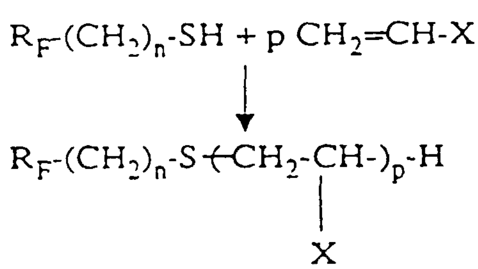

with $R_F$, n, p and X having the same definition as in formula (I), under an inert atmosphere in the presence of a free-radical initiator.

**5.** Process for the preparation of the novel compounds corresponding to the formula:

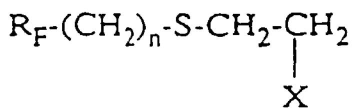

in which:

$R_F$ is a linear or branched $C_4$-$C_{10}$, preferably $C_6$-$C_8$, perfluoroalkyl radical or a mixture of linear or branched $C_4$-$C_{10}$, preferably $C_6$-$C_8$, perfluoroalkyl radicals;
n is an integer between 0 and 4, preferably equal to 2;
X is an aromatic group which imparts to the compound of formula (I) absorption of ultraviolet in the wavelength region between 280 and 400 nm,

characterized in that it consists of a radical addition, in a solvent medium, of a mercaptan containing a perfluoro chain to one or more molecules which absorb ultraviolet radiation in the chosen wavelength region and which contain a group containing a reactive double bond, according to the following reaction scheme:

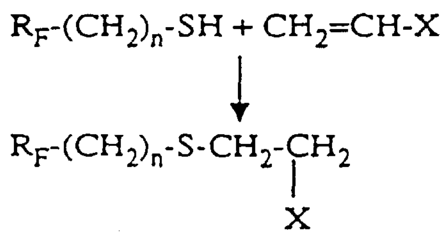

with $R_F$, n and X having the same meaning as above, under an inert atmosphere in the presence of a basic catalyst.

**6.** Use of the compounds of formula (I):

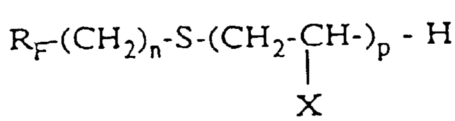

(I)

in which:

$R_F$ is a linear or branched $C_4$-$C_{10}$, preferably $C_6$-$C_8$, perfluoroalkyl radical or a mixture of linear or branched $C_4$-$C_{10}$, preferably $C_6$-$C_8$, perfluoroalkyl radicals;
n is an integer between 0 and 4, preferably equal to 2;
p is an integer or statistical number between 1 and 10, preferably between 1 and 5; and
X is an aromatic group which imparts to the compound of formula (I) absorption of ultraviolet in the wavelength region between 280 and 400 nm;

as sunscreens in the UV-A and UV-B region.

**7.** Cosmetic composition which screens out ultraviolet rays, characterized in that it contains, in a cosmetically acceptable support, an effective amount of at least one sunscreen with a perfluorocarbon chain, of formula (I):

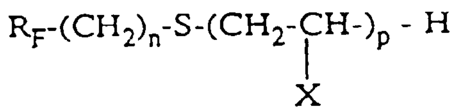 (I)

in which:

- $R_F$ is a linear or branched $C_4$-$C_{10}$, preferably $C_6$-$C_8$, perfluoroalkyl radical or a mixture of linear or branched $C_4$-$C_{10}$, preferably $C_6$-$C_8$, perfluoroalkyl radicals;
- n is an integer between 0 and 4, preferably equal to 2;
- p is an integer or statistical number between 1 and 10, preferably between 1 and 5; and
- X is an aromatic group which imparts to the compound of formula (I) absorption of ultraviolet in the wavelength region between 280 and 400 nm.

**8.** Cosmetic composition according to Claim 7, characterized in that, in formula (I), X denotes the group

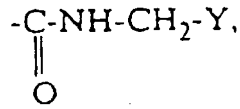

Y representing an aromatic radical which absorbs ultraviolet radiation in the wavelength region ranging from 280 to 400 nm.

**9.** Cosmetic composition according to Claim 8, characterized in that Y is chosen from:

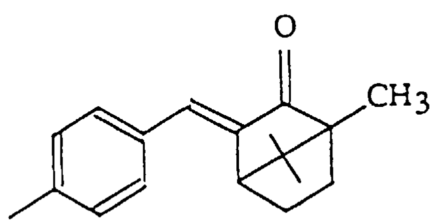 (II)

and

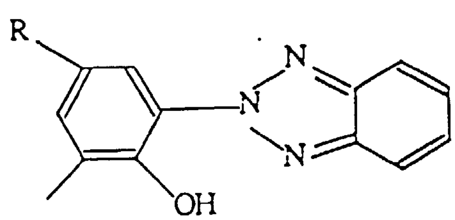 (III)

R denoting a methyl or tert-octyl radical.

**10.** Cosmetic composition according to any one of Claims 7 to 9, characterized in that the cosmetically acceptable support contains at least one fatty phase or an organic solvent.

**11.** Cosmetic composition according to any of Claims 7 to 10, characterized in that the compound of formula (I) is present in the composition in proportions of between 0.1 and 15% by weight relative to the total weight of the

composition.

12. Cosmetic composition according to any one of Claims 7 to 11, characterized in that it is in the form of a lotion, a gel, a cream, a milk, a powder or a solid stick and is optionally packaged as an aerosol and is in the form of a foam or a spray.

13. Cosmetic composition according to any one of Claims 7 to 12, characterized in that it also contains at least one adjuvant chosen from fatty substances, organic solvents, silicones, thickeners, softeners, UV-A, UV-B or broad-band sunscreens, anti-foaming agents, moisturizers, fragrances, preserving agents, surfactants, fillers, sequestering agents, anionic, cationic, nonionic or amphoteric polymers or mixtures thereof, propellants, basifying or acidifying agents, dyes and pigments.

14. Cosmetic composition according to any one of Claims 7 to 13, which constitutes a composition for protecting the human epidermis against ultraviolet rays or an antisun composition, characterized in that it is in the form of a suspension or dispersion in solvents or fatty substances, an emulsion, a vesicle dispersion, an ointment, a gel, a solid stick or an aerosol foam.

15. Cosmetic composition according to any one of Claims 7 to 13, for protecting the hair against ultraviolet rays, characterized in that it is in the form of a shampoo, a lotion, a gel or a composition to be rinsed out, to be applied before or after shampooing, before or after dyeing or bleaching, before, during or after permanent-waving or straightening of the hair, or in the form of a styling or treating lotion or gel, a blow-drying or hairstyling lotion or gel, a hair lacquer or a composition for the permanent-waving, straightening, dyeing or bleaching of the hair.

16. Cosmetic composition according to any one of Claims 7 to 13, which constitutes a product for making up the eyelashes, the eyebrows, the skin, the nails or the hair, characterized in that it is in the form of a skin-treatment cream, a foundation, a lipstick, an eyeshadow, a blusher, an eyeliner, a mascara or a colouring gel, and is in anhydrous or aqueous and solid or pasty form.

17. Process for protecting the human epidermis and the hair against ultraviolet radiation, characterized in that it consists in applying an effective amount of a sunscreen containing a perfluorocarbon chain, as defined in one of Claims 1 to 3, to the skin or the hair.